# EUROPÄISCHE PATENTANMELDUNG

(11) **EP 3 854 773 A1**
(43) Veröffentlichungstag der Anmeldung: **28.07.2021**
(21) Anmeldenummer: 20153907.9
(22) Anmeldetag: 27.01.2020
(51) Int. Cl.: C07C 41/50, C07C 41/56, C07C 43/30

(54) **VERFAHREN ZUR ERZEUGUNG VON OXYMETHYLENETHER**

(71) Anmelder: Friedrich-Alexander-Universität Erlangen-Nürnberg, 91054 Erlangen (DE); OxFA GmbH, 96110 Scheßlitz (DE)
(72) Erfinder: ALBERT, Jakob, 91054 Erlangen (DE); BUKOWSKI, Anna, 91052 Erlangen (DE); VOSS, Dorothea, 91056 Erlangen (DE)
(74) Vertreter: Dr. Gassner & Partner mbB

(57) **Zusammenfassung**

Die Erfindung betrifft ein Verfahren zur Erzeugung von Oxymethylenether der allgemeinen Formel CH₃O-(CH₂O)ₘ-CH₃, wobei 1 ≤ m ≤ 10, wobei in einer katalytischen Reaktion molekularer Sauerstoff oder ein Sauerstoff enthaltendes Oxidationsmittel und Methanol, Formaldehyd und/oder Methylformiat in einer Lösung als Edukte eingesetzt und mittels einer Vanadium in der Oxidationsstufe +IV oder +V enthaltenden Vanadium-Sauerstoff-Verbindung oder eines Salzes davon als Katalysator in der Lösung umgesetzt werden, wobei der bei der katalytischen Reaktion reduzierte Katalysator durch Oxidation mittels dem molekularen Sauerstoff oder dem Sauerstoff enthaltenden Oxidationsmittel wieder in seinen Ausgangszustand versetzt wird.

## Beschreibung

Die Erfindung betrifft ein Verfahren zur Erzeugung von Oxymethylenether (OME) der allgemeinen Formel CH₃O-(CH₂O)ₘ-CH₃ mit 1 ≤ m ≤ 10 in einer katalytischen Reaktion.

OMEs können als Additiv Dieselkraftstoff zugesetzt werden, um die Rußbildung in einem Dieselmotor zu vermindern.

Aus der DE 2 163 907 ist ein Verfahren zur Herstellung von Polyoxymethylendialkylethern bekannt, bei dem ein niedrigmolekularer Alkohol mit einer Formaldehyd-bildenden Substanz in Gegenwart eines sauren Katalysators umgesetzt wird.

Aus der DE 10 2005 027 702 A1 ist ein Verfahren zur Herstellung von Polyoxymethylendimethylethern aus Methanol und Formaldehyd bekannt. Dabei wird das dabei entstehende Gemisch, enthaltend Formaldehyd, Wasser, Methylenglykol, Polyoxymethylenglykole, Methanol, Hemiformale, Methylal und Polyoxymethylenglykoldimethylether durch Destillation aufgearbeitet. Nachteilig bei diesem Verfahren sowie dem aus der DE 2 163 907 bekannten Verfahren ist, dass ein komplexes Gemisch von Reaktionsprodukten entsteht und die Gewinnung von Polyoxymethylendimethylethern daraus mit erheblichem Aufwand verbunden ist.

Aus der WO 2006/045506 A1 ist ein Verfahren zur Herstellung von Polyoxymethylendimethylether bekannt, bei dem Methylal und Trioxan in Gegenwart eines sauren Katalysators umgesetzt werden. Das Verfahren zeichnet sich dadurch aus, dass die durch Methylal, Trioxan und/oder den Katalysator in das Reaktionsgemisch eingebrachte Wassermenge weniger als 1 Gew.-%, bezogen auf das Reaktionsgemisch, beträgt. Bei dem Verfahren wird zur Herstellung von längerkettigen Polyoxymethylendimethylethern Dimethoxymethan eingesetzt.

Aus der DE 10 2014 112 021 A1 ist ein Verfahren zur Herstellung von Oxymethylendialkylethern und deren direkte Verwendung als Kraftstoffadditive bekannt. Bei dem Verfahren wird ein Alkohol und/oder eine Carbonsäure mit einem Aldehyd und/oder einem Keton in Anwesenheit eines sauren Katalysators umgesetzt. Während der Reaktion oder anschließend wird durch Zugabe oder Entstehung eines Extraktionsmittels eine wässrige und eine organische Phase erzeugt und anschließend die organische Phase entnommen.

Allen oben genannten Verfahren auf Basis von Methanol ist gemein, dass sie eine geringe Selektivität besitzen und damit zu komplexen Produktgemischen führen, die zahlreiche unerwünschte Komponenten enthalten können.

Aus Tang, Z. et al., ChemSusChem 2014, 7, Seiten 1557 bis 1567 ist eine durch Vanadyl-Kationen katalysierte Umsetzung von Zellulose zu Ameisensäure und Milchsäure bekannt. Insbesondere wird die Verwendung von VOSO₄ als Katalysator zur Umsetzung von Glucose zu Ameisensäure und zu Milchsäure offenbart. Durch die Bildung von CO₂ bei dieser Umsetzung ist die Ausbeute von Ameisensäure auf etwas über 50% beschränkt. Es wurde jedoch gefunden, dass das Zusetzen von Methanol oder Ethanol zu dem Reaktionssystem die Bildung von CO₂ während der Umsetzung von Glucose unter aeroben Bedingungen unterdrückt und die Ausbeute an Ameisensäure dadurch auf 70% bis 75% erhöht werden konnte.

Aufgabe der vorliegenden Erfindung ist es, ein alternatives Verfahren zur Herstellung von Oxymethylenether anzugeben. Insbesondere soll das Verfahren Oxymethylenether mit hoher Selektivität ohne eine große Zahl unerwünschter Nebenprodukte liefern.

Erfindungsgemäß wird die Aufgabe durch die Merkmale des Patentanspruchs 1 gelöst. Zweckmäßige Ausgestaltungen ergeben sich aus den Merkmalen der Patentansprüche 2 bis 15.

Erfindungsgemäß ist ein Verfahren zur Erzeugung von Oxymethylenether der allgemeinen Formel CH₃O-(CH₂O)ₘ-CH₃ in einem Flüssigphasenprozess vorgesehen, wobei 1 ≤ m ≤ 10, wobei in einer katalytischen Reaktion, insbesondere ausschließlich, molekularer Sauerstoff oder ein Sauerstoff enthaltendes Oxidationsmittel und Methanol, Formaldehyd und/oder Methylformiat in einer Lösung als Edukte eingesetzt und mittels einer Vanadium in der Oxidationsstufe +IV oder +V enthaltenden Vanadium-Sauerstoff-Verbindung oder eines Salzes davon als Katalysator in der Lösung umgesetzt werden, wobei der bei der katalytischen Reaktion reduzierte Katalysator durch Oxidation mittels dem molekularen Sauerstoff oder dem Sauerstoff enthaltenden und diesen Sauerstoff an den reduzierten Katalysator abgebenden Oxidationsmittel wieder in seinen Ausgangszustand versetzt wird.

Der dabei erzeugte Oxymethylenether kann, insbesondere durch eine Extraktion oder mittels eines sonstigen bekannten Trennverfahrens, insbesondere unter Verwendung einer semipermeablen Membran, aus der Lösung abgesondert werden.

Bei einer Ausgestaltung dieses Verfahrens werden in der katalytischen Reaktion, insbesondere ausschließlich, der molekulare Sauerstoff oder das Sauerstoff enthaltendes Oxidationsmittel und Methanol in der Lösung als Edukte eingesetzt.

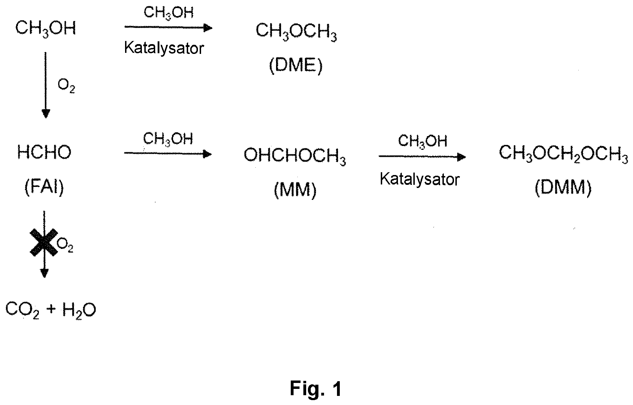

Die Erfinder haben festgestellt, dass durch die Verwendung von Methanol, Formaldehyd und/oder Methylformiat und molekularem Sauerstoff oder einem Sauerstoff enthaltenden Oxidationsmittel als Edukte direkt Dimethoxymethan, d. h. CH₃O-(CH₂O)ₘ-CH₃ mit m = 1, in flüssiger Phase erzeugt werden kann, selbst wenn ausschließlich diese Edukte verwendet werden. Die Erfinder nehmen an, dass bei der Umsetzung von Methanol durch teilweise partielle Oxidation des Methanols in-situ Formaldehyd (FAI) entsteht. Weiterhin nehmen sie an, dass das Formaldehyd anschließend mit weiterem Methanol zu Methoxymethanol (MM) und dieses in einem letzten Reaktionsschritt mit weiterem Methanol in Gegenwart des Katalysators zu Dimethoxymethan (DMM) reagiert. Eine vollständige Oxidation des Formaldehyds zu CO₂ und H₂O erfolgt überraschenderweise nicht oder zumindest nicht in wesentlichem Umfang. Als Nebenprodukt entsteht lediglich Dimethylether (DME), wobei das Verhältnis von DMM zu DME durch Wahl einer verhältnismäßig niedrigen Reaktionstemperatur, beispielsweise im Bereich zwischen 70 °C und 90 °C, deutlich zum DMM hin verschoben wird. Ein entsprechendes Reaktionsschema ist in Fig. 1 dargestellt. Das DMM kann durch eine Extraktion oder mittels eines sonstigen bekannten Trennverfahrens, insbesondere unter Verwendung einer semipermeablen Membran, aus der Lösung abgesondert werden.

Der Katalysator kann ein Polyoxometallat-Ion der allgemeinen Formel [PMoₓV_{y}O₄₀]ⁿ⁻, wobei 6 ≤ x ≤ 11, 1 ≤ y ≤ 6 und x + y = 12, [WₓV_{y}O₁₉]ⁿ⁻, wobei x + y = 6, 3 ≤ x ≤ 5 und 1 ≤ y ≤ 3 oder [P₂WₓV_{y}O₆₂]ⁿ⁻, wobei x + y = 18, 12 ≤ x ≤ 17 und 1 ≤ y ≤ 6 oder ein VO²⁺ enthaltendes Salz, insbesondere VOSO₄, oder ein [VO₃]⁻ enthaltendes Salz, insbesondere NH₄VO₃, sein, wobei n, x und y jeweils eine ganze Zahl ist. Der Wert von n ergibt sich dabei aus den Teilladungen der im Katalysator enthaltenen Elemente. Bei [PMoₓV_{y}O₄₀]ⁿ⁻ beispielsweise ist 3 < n < 10. Als gut geeignet hat sich das Polyoxometallat-Ion [PMoₓV_{y}O₄₀]ⁿ⁻, insbesondere [PMo₇V₅O₄₀]⁸⁻ (HPA-5), erwiesen. Wegen der von den Ionen gebildeten spezifischen Strukturen wird [PMoₓV_{y}O₄₀]ⁿ⁻ auch als Keggin-Ion, [WₓV_{y}O₁₉]ⁿ⁻ als Lindqvist-Ion und [P₂WₓV_{y}O₆₂]ⁿ⁻ als Wells-Dawson-Ion bezeichnet.

Die Oxidation mittels dem molekularen Sauerstoff kann mittels dem molekularen Sauerstoff als reinem Gas oder in einem den molekularen Sauerstoff enthaltenden Gasgemisch, insbesondere Luft oder synthetische Luft, erfolgen. Bei synthetischer Luft handelt es sich im Allgemeinen um ein aus Sauerstoff und Stickstoff bestehendes Gasgemisch, bei welchem der Sauerstoffanteil im Bereich von 19,5 Vol.-% bis 21,5 Vol.-% liegt.

Das den Sauerstoff enthaltende Oxidationsmittel kann ein Peroxid, insbesondere H₂O₂, oder N₂O, sein. Die Oxidation mittels dem molekularen Sauerstoff kann bei einem Sauerstoffdruck - im Falle von Sauerstoff als reinem Gas - oder einem Sauerstoffpartialdruck - im Falle eines Gasgemischs - im Bereich von 1 bar bis 250 bar, insbesondere 1 bar bis 120 bar, insbesondere 1 bar bis 80 bar, insbesondere 1 bar bis 50 bar, insbesondere 1 bar bis 30 bar, insbesondere 5 bar bis 20 bar, insbesondere 5 bar bis 10 bar, erfolgen. Zur Oxidation kann die Lösung, beispielsweise in einem statischen Mischer oder durch heftiges Rühren, mit dem molekularen Sauerstoff beaufschlagt werden.

Die Reaktion zur Erzeugung von Oxymethylenether kann durch eine Erhöhung der Temperatur beschleunigt werden. Bei einer Ausgestaltung des Verfahrens wird die katalytische Reaktion bei einer Temperatur von höchstens 150 °C, insbesondere in einem Bereich von 70 °C bis 150 °C, durchgeführt. Um möglichst wenig des Nebenprodukts Dimethylether im Verhältnis zum gewünschten Oxymethylenether entstehen zu lassen, hat es sich als günstig erwiesen, die katalytische Reaktion bei einer Temperatur im Bereich von 70 °C bis 90 °C durchzuführen.

Für das Verfahren ist es günstig, wenn die Lösung, insbesondere am Begin der katalytischen Reaktion, möglichst wenig Wasser, insbesondere weniger als 5 Gew.-% Wasser, insbesondere weniger als 1 Gew.-% Wasser, enthält. Bei der katalytischen Reaktion kann/können das Methanol und/oder das Formaldehyd und/oder das Methylformiat als, insbesondere einziges, Lösungsmittel bzw. Lösungsmittelgemisch in der Lösung eingesetzt werden. Das Methanol, das Formaldehyd und/oder das Methylformiat wäre(n) dann gleichzeitig Edukt(e) und Lösungsmittel. Anfänglich können in der mit Sauerstoff beaufschlagten Lösung also lediglich Methanol, das Formaldehyd und/oder das Methylformiat und der Katalysator enthalten sein. Bei einer Ausgestaltung des Verfahrens wird bei der katalytischen Reaktion neben dem molekularen Sauerstoff oder dem Sauerstoff enthaltenden Oxidationsmittel, insbesondere nur, das Methanol als Edukt und, insbesondere einziges, Lösungsmittel in der Lösung eingesetzt.

Bei einer Ausgestaltung des Verfahrens ist die Kettenlänge des zu erzeugenden Oxymethylenethers so gewählt, dass 1 ≤ m ≤ 6 ist. Bei dem Oxymethylenether kann es sich um Dimethoxymethan handeln. In diesem Fall ist m = 1. Dimethoxymethan ist der bei dem Verfahren zuerst entstehende Oxymethylenether. Eine durch den Katalysator bewirkte weitere Umsetzung des Dimethoxymethans kann unterbunden werden, indem das Dimethoxymethan und der Katalysator, insbesondere durch Extraktion oder mittels eines Trennverfahrens unter Verwendung einer semipermeablen Membran, voneinander getrennt werden. Dazu kann entweder der Katalysator oder das Dimethoxymethan aus der Lösung entfernt werden. Alternativ kann der Katalysator in seiner Wirkung auch inaktiviert werden. Bei einem Polyoxometallat als Katalysator kann dies beispielsweise dadurch erfolgen, dass die Lösung alkalisch gemacht wird, beispielsweise indem, insbesondere durch Zusatz eines Hydroxids, der pH-Wert auf einen Wert größer als 8, insbesondere einen Wert größer als 10, insbesondere einen Wert größer als 12, insbesondere einen Wert größer als 13,5, insbesondere einen Wert von 14, eingestellt wird. Polyoxometallate sind bei solchen pH-Werten nicht stabil und werden irreversibel inaktiviert.

Das Dimethoxymethan kann aufgrund seines niedrigen Siedepunkts von 42 °C gut durch Destillation aus dem Reaktionsansatz abgesondert werden. Alternativ kann es durch Extraktion oder mittels eines Trennverfahrens unter Verwendung einer semipermeablen Membran von der Lösung abgesondert werden.

Eine Extraktion von Dimethoxymethan kann mittels eines aus der Tabelle auf Seite 9 der DE 10 2014 112 021 A1 bekannten und für die Extraktion von Dimethoxymethan (mit "X" gekennzeichnete Spalte der Tabelle) geeigneten Extraktionsmittels, welches eine Phasenbildung bewirkt (mit "P" gekennzeichnete Spalte der Tabelle), erfolgen. Dabei kann es sich z.B. um Nitrobenzol, Benzol, Dichlormethan, Ölsäuremethylester oder Diesel handeln.

Soll ein Oxymethylenether erzeugt werden, bei dem m größer als 1 ist, kann die Lösung dazu nach einer Bildung von Dimethoxymethan, insbesondere bei einem Sauerstoffpartialdruck unter 1 bar, insbesondere bei Atmosphärendruck, und/oder einer Temperatur unter 70 °C, insbesondere bei einer Temperatur zwischen 20 °C und 35 °C, weiter inkubiert werden, bis m einen zuvor gewählten Wert erreicht hat. Die Analyse der Lösung zur Bestimmung der Kettenlänge des gebildeten Oxymethylenethers kann beispielsweise mittels Gaschromatografie (GC) unter Verwendung entsprechender Vergleichssubstanzen erfolgen. Die Erfinder haben festgestellt, dass die Erzeugung von Oxymethylenether mit einer größeren Kettenlänge als derjenigen von Dimethoxymethan in der Lösung, katalysiert durch den Katalysator, erfolgt und dass dazu weder ein erhöhter Sauerstoffpartialdruck noch eine erhöhte Temperatur erforderlich sind. Zur Beschleunigung der Kettenverlängerung kann der Lösung auch vor oder nach der Bildung des Dimethoxymethans Trioxan zugesetzt werden. Die Reaktion kann dann beispielsweise bei 25 °C und Atmosphärendruck erfolgen. Als günstig hat sich eine Reaktion bei einem Druck von 1 bar bis 20 bar, insbesondere 1 bar bis 10 bar, und bei einer Temperatur von 50 °C bis 200 °C, insbesondere 60 °C bis 130 °C, erwiesen. Die Reaktionsdauer kann beispielsweise im Bereich von 20 Minuten bis 120 Minuten liegen.

Bei einer Ausgestaltung des Verfahrens werden in der katalytischen Reaktion ausschließlich der molekulare Sauerstoff oder das Sauerstoff enthaltendes Oxidationsmittel, Trioxan und Methanol, Formaldehyd und/oder Methylformiat in der Lösung als Edukte eingesetzt. Bei einer weiteren Ausgestaltung des Verfahrens werden in der katalytischen Reaktion ausschließlich der molekulare Sauerstoff oder das Sauerstoff enthaltendes Oxidationsmittel, Trioxan und Methanol in der Lösung als Edukte eingesetzt.

Nachfolgend wird die Erfindung anhand von Ausführungsbeispielen näher erläutert.

Fig. 1 zeigt ein Reaktionsschema der erfindungsgemäßen Umsetzung von Methanol.

### 1. Ausführungsbeispiel:

In einem ersten Ausführungsbeispiel wurden entweder 10 g Methanol als Lösungsmittel und Edukt bzw. Substrat eingesetzt oder jeweils 1 mmol Methylformiat (MF) oder Formaldehyd (FAl) als Substrat in 10 g Methanol als Lösungsmittel eingesetzt. Als Katalysator wurde 0,1 mmol des Polyoxometallat-Ions [PMo₇V₅O₄₀]⁸⁻(= HPA-5) zugesetzt. Die resultierende Lösung wurde für 24 Stunden mit 1000 Umdrehungen pro Minute gerührt, dabei bei einer Temperatur von 90 °C gehalten und Sauerstoff mit einem Sauerstoffpartialdruck von 20 bar ausgesetzt. Die Ergebnisse sind in der nachfolgenden Tabelle zusammengefasst:

| Substrat | W_{H₂O,Reinstoff} / Gew.-% | W_{H₂O,nach} / Gew.-% | DME | FAl | MM | DMM | FA | MF | Y_{CO₂/CO} / % |
|---|---|---|---|---|---|---|---|---|---|
| Methanol (MeOH) | 0,05 | 2,1 | x | - | - | x | - | - | - / - |
| Methylformiat (MF) | 0,15 | 1,8 | x | - | - | x | - | x | - / - |
| Formaldehyd (FAl) | > 50 W_{H₂O,vor} = 0,5 | 2,4 | x | - | - | x | - | - | 0,6/- |

Die Spalte w_{H₂O,Reinstoff} gibt den gewichtsprozentualen Anteil an Wasser in dem jeweiligen Substrat an. Die Spalte w_{H₂O,nach} gibt den gewichtsprozentualen Anteil an Wasser in der Lösung nach Ablauf der Reaktion an. w_{H₂O,vor} gibt bei Formaldehyd den gewichtsprozentualen Anteil an Wasser in der Lösung vor der Reaktion an. Das Vorhandensein der einzelnen Reaktionsprodukte wurde mittels Nuklearmagnetischer Resonanzspektroskopie (NMR) bestimmt. Sofern kein Reaktionsprodukt aufgefunden wurde, wurde dies mit einem "-" gekennzeichnet, ansonsten mit einem "x". Dabei haben die Abkürzungen die folgenden Bedeutungen:
- DME:: Dimethylether
- FAI:: Formaldehyd
- MM:: Methoxymethanol
- DMM:: Dimethoxymethan
- FA:: Ameisensäure
- MF:: Methylformiat

Aus der Tabelle ist ersichtlich, dass bei der Verwendung von Methanol als Ausgangsstoff mit hoher Selektivität nur der Oxymethylenether Dimethoxymethan und das Nebenprodukt Dimethylether und kein CO₂ gebildet wird. Auch wenn Formaldehyd oder Methylformiat eingesetzt wird, werden nur der Oxymethylenether Dimethoxymethan und das Nebenprodukt Dimethylether gebildet. Beim Einsatz von Methylformiat war nach Abschluss der Reaktion noch ein Teil des eingesetzten Methylformiats im Ansatz nachweisbar.

### 2. Ausführungsbeispiel:

Für eine alternative Synthese von OMEs mit einer Kettenlänge von 2 bis 6 wurde ein Molverhältnis von Trioxan zu Dimethoxymethan (Methylal) von 0,33 und der Katalysator in einer Menge von 1 Gew.-% in Bezug auf Trioxan verwendet. Die Reaktionen wurden in einem Glaskolben bei Atmosphärendruck und einer Temperatur von 25 °C durchgeführt. Vor dem Versuch wurde sowohl das Reaktionsgefäß als auch das Methylal getrocknet. Nach Zugabe des Katalysators wurde die resultierende Lösung für 60 Minuten bei 800 Umdrehungen pro Minute gerührt. Eine Analyse der Reaktionsprodukte erfolgte mittels eines Gaschromatografen.

### 3. Ausführungsbeispiel:

Für eine weitere alternative Synthese von OMEs mit einer Kettenlänge von 2 bis 6 wurde ein Edelstahlreaktor verwendet. Dieser wurde mit Dimethoxymethan, Trioxan und dem Katalysator, befüllt. Das molare Verhältnis von Dimethoxymethan zu Trioxan wurde zwischen 2,5:1 und 1:2 variiert und es wurde 2 Gew.-% an Katalysator bezogen auf die Ausgangstoffe eingesetzt. Die Reaktionstemperatur wurde in einem Bereich von 70 °C bis 130 °C eingestellt. Die Reaktionsdauer wurde in einem Bereich von 20 Minuten bis 120 Minuten gewählt. Es wurde ein Reaktionsdruck von 10 bar und eine Rührgeschwindigkeit von 300 Umdrehungen pro Minute eingestellt.

Die Ausführungsbeispiele 2 und 3 zeigten, dass ausgehend von dem bei dem erfindungsgemäßen Verfahren gebildeten Dimethoxymethan unter Zusatz von Trioxan Oxymethylenether mit einer Kettenlänge von 2 bis 6, d. h. Oxymethylenether bei denen 2 ≤ m ≤ 6 gemäß der oben angegebenen allgemeinen Formel ist, erhalten werden können.

## Patentansprüche

1. Verfahren zur Erzeugung von Oxymethylenether der allgemeinen Formel CH₃O-(CH₂O)ₘ-CH₃, wobei 1 ≤ m ≤ 10, wobei in einer katalytischen Reaktion molekularer Sauerstoff oder ein Sauerstoff enthaltendes Oxidationsmittel und Methanol, Formaldehyd und/oder Methylformiat in einer Lösung als Edukte eingesetzt und mittels einer Vanadium in der Oxidationsstufe +IV oder +V enthaltenden Vanadium-Sauerstoff-Verbindung oder eines Salzes davon als Katalysator in der Lösung umgesetzt werden, wobei der bei der katalytischen Reaktion reduzierte Katalysator durch Oxidation mittels dem molekularen Sauerstoff oder dem Sauerstoff enthaltenden Oxidationsmittel wieder in seinen Ausgangszustand versetzt wird.

2. Verfahren nach Anspruch 1, wobei der in der katalytischen Reaktion erzeugte Oxymethylenether, insbesondere durch eine Extraktion oder mittels eines Trennverfahrens unter Verwendung einer semipermeablen Membran, aus der Lösung abgesondert wird.

3. Verfahren nach einem der vorhergehenden Ansprüche, wobei in der katalytischen Reaktion ausschließlich der molekulare Sauerstoff oder das den Sauerstoff enthaltende Oxidationsmittel und Methanol, Formaldehyd und/oder Methylformiat als Edukte eingesetzt werden.

4. Verfahren nach einem der vorhergehenden Ansprüche, wobei der Katalysator ein Polyoxometallat-Ion der allgemeinen Formel [PMoₓV_{y}O₄₀]ⁿ⁻, wobei 6 ≤ x ≤ 11, 1 ≤ y ≤ 6 und x + y = 12, [WₓV_{y}O₁₉]ⁿ⁻, wobei x + y = 6, 3 ≤ x ≤ 5 und 1 ≤ y ≤ 3 oder [P₂WₓV_{y}O₆₂]ⁿ⁻, wobei x + y = 18, 12 ≤ x ≤ 17 und 1 ≤ y ≤ 6 oder ein VO²⁺ enthaltendes Salz, insbesondere VOSO₄, oder ein [VO₃]⁻ enthaltendes Salz, insbesondere NH₄VO₃, ist, wobei n, x und y jeweils eine ganze Zahl ist.

5. Verfahren nach einem der vorhergehenden Ansprüche, wobei der molekulare Sauerstoff in einem den molekularen Sauerstoff enthaltenden Gasgemisch, insbesondere Luft, enthalten ist und das den Sauerstoff enthaltende Oxidationsmittel ein Peroxid, insbesondere H₂O₂, oder N₂O, ist.

6. Verfahren nach einem der vorhergehenden Ansprüche, wobei die Oxidation mittels dem molekularen Sauerstoff bei einem Sauerstoffdruck oder Sauerstoffpartialdruck im Bereich von 1 bar bis 50 bar, insbesondere 1 bar bis 30 bar, insbesondere 5 bar bis 20 bar, erfolgt.

7. Verfahren nach einem der vorhergehenden Ansprüche, wobei die katalytische Reaktion bei einer Temperatur von höchstens 150 °C, insbesondere in einem Bereich von 70 °C bis 150 °C, insbesondere in einem Bereich von 70 °C bis 90 °C, durchgeführt wird.

8. Verfahren nach einem der vorhergehenden Ansprüche, wobei die Lösung weniger als 5 Gew.-% Wasser, insbesondere weniger als 1 Gew.-% Wasser, enthält.

9. Verfahren nach einem der vorhergehenden Ansprüche, wobei das Methanol, das Formaldehyd und/oder das Methylformiat bei der katalytischen Reaktion auch als, insbesondere einziges, Lösungsmittel oder Lösungsmittelgemisch eingesetzt wird/werden.

10. Verfahren nach einem der vorhergehenden Ansprüche, wobei 1 ≤ m ≤ 6.

11. Verfahren nach einem der vorhergehenden Ansprüche, wobei der Oxymethylenether Dimethoxymethan ist.

12. Verfahren nach Anspruch 11, wobei eine durch den Katalysator bewirkte weitere Umsetzung des Dimethoxymethans unterbunden wird, indem das Dimethoxymethan und der Katalysator, insbesondere durch Extraktion oder mittels eines Trennverfahrens unter Verwendung einer semipermeablen Membran, voneinander getrennt werden.

13. Verfahren nach Anspruch 11 oder 12, wobei das Dimethoxymethan durch Destillation, Extraktion oder mittels eines Trennverfahrens unter Verwendung einer semipermeablen Membran von der Lösung abgesondert wird.

14. Verfahren nach einem der Ansprüche 1 bis 10, wobei ein Oxymethylenether mit m > 1 gebildet wird, indem die Lösung nach einer Bildung von Dimethoxymethan, insbesondere bei einem Sauerstoffpartialdruck unter 1 bar und/oder einer Temperatur unter 70 °C, weiter inkubiert wird, bis m einen gewählten Wert erreicht hat.

15. Verfahren nach Anspruch 14, wobei der Lösung Trioxan zugesetzt wird.
